# EUROPEAN PATENT SPECIFICATION

(11) **EP 4 378 843 B1**
(45) Date of publication and mention of the grant of the patent: **15.10.2025**
(21) Application number: 23208887.2
(22) Date of filing: 09.11.2023
(51) Int. Cl.: B65B 55/02, B65B 55/04, A61L 2/20, A61L 2/10, A61L 2/04, A61L 2/18

(54) **DEVICE FOR REDUCTION OF MICROORGANISMS ON WEB MATERIAL**
VORRICHTUNG ZUR REDUKTION VON MIKROORGANISMEN AUF EINEM BAHNMATERIAL
DISPOSITIF DE RÉDUCTION DE MICRO-ORGANISMES SUR UN MATÉRIAU EN BANDE

(30) Priority: 30.11.2022 EP 22210406
(43) Date of publication of application: 05.06.2024
(73) Proprietor: Tetra Laval Holdings & Finance S.A., 1009 Pully (CH)
(72) Inventor: DELÉN, Anders, 22186 Lund (SE)
(74) Representative: Tetra Pak Patent Attorneys

(56) References cited:
- EP-B1- 0 836 854
- CN-A- 108 951 071
- US-A1- 2006 284 111

## Description

### Technical Field

The present disclosure relates to a device for reducing presence of microorganisms on a web material, in particular a web material for use in production of packages.

### Background Art

It is vital to reduce presence of microorganisms during production of certain types of packages, for example packages that contain food products. This may be achieved by disinfection or sterilization.

One type of package is manufactured from a web material, which is cut and shaped into a package and filled with a food product. The web material may be a fiberbased laminate, for example comprising a core layer of paper or paperboard and one or more barrier layers of plastic. The packages are manufactured in a production line that includes one or more conventional filling machines. The web material is typically provided to the production line on rolls.

Disinfection or sterilization may be performed at different stages in the production line. It is common practice to disinfect the incoming web material to prevent contamination of downstream equipment in the production line. The disinfection may be performed by feeding the web material through a disinfection device or unit, in which the web material is subjected to disinfection. Ports on the disinfection device may be provided with flexing seals that engage the passing web material so that the disinfection is performed in an effectively closed space within the housing. Alternatively or additionally, a corresponding sterilization device may be arranged in the production line to perform a sterilization of the web material.

Industrial production of packages is automated and designed for high-volume production. Standstill of production for service and maintenance is costly. When the web material on a roll has been consumed, web material from another roll needs to be introduced into the production line via the disinfection/sterilization device. The time for switching between rolls should be minimized.

### Summary

It is an objective to at least partly overcome one or more of the above-identified limitations of the prior art.

One such objective is to facilitate switching of web material in a production line that comprises equipment for reducing presence of microorganisms on the web material. Herein, reducing presence of microorganisms means reducing presence of microorganisms that are alive, and may comprise both disinfection and sterilization.

One or more of these objectives, as well as further objectives that may appear from the description below, are at least partly achieved by a device for reducing presence of microorganisms on a web material, and a system for manufacturing of packages according to the independent claims, embodiments thereof being defined by the dependent claims.

A first aspect relates to a device for reducing presence of microorganisms on a web material. The device comprises a housing that defines an interior space; and an inlet port and an outlet port, which are arranged on the housing to allow the web material to move along a travel path through the interior space between the inlet port and the outlet port. The inlet and outlet ports are elongated and comprise a respective elongated seal for flexing engagement with the web material. The device further comprises an arrangement for reducing the presence of microorganisms on the web material within the interior space. The device further comprises a moveable element comprising a support surface. The moveable element is arranged within the housing for movement into a first position, in which the support surface is arranged along the travel path so as to direct a leading end of the web material from the inlet port towards the outlet port, and a second position, in which the support surface is spaced from the travel path.

In some embodiments, the support surface, when the moveable element is in the second position, is spaced from the travel path so as provide said arrangement, which is fixedly arranged in the housing, unobscured access to the web material to reduce the presence of microorganisms on the web material.

In some embodiments, the device further comprises a sensor, which is arranged to generate a signal indicative of whether the moveable element is in the first position or the second position.

In some embodiments, the device further comprises a control unit, which is configured to operate said arrangement based on the signal from the sensor.

In some embodiments, the control unit is configured to disable said arrangement when the moveable element is in the first position and enable said arrangement when the moveable element is in the second position.

In some embodiments, the support surface is flat.

In some embodiments, the support surface, when the moveable element is in the first position, is parallel to the inlet and outlet ports.

In some embodiments, the outlet port comprises an access opening in the housing, and the elongated seal, which is arranged to cover the access opening, wherein the access opening and/or the elongated seal defines a bounding surface of the outlet port inside the housing, and wherein the support surface, when the moveable element is in the first position, is inclined and/or shifted in relation to the bounding surface, based on a predefined curvature of the web material, so as to direct the leading end of the web material to outlet port.

In some embodiments, a front end of the support surface is located at the outlet port when the moveable element is in the first position, and wherein a first distance between the front end and the bounding surface, perpendicular to the bounding surface, is in a range of 0-50 mm, and preferably in a range of 0.5-40 mm.

In some embodiments, the elongated seal defines an elongated slit for receiving the web material, and the front end of the support surface, when the moveable element is in the first position, is located a second distance from the elongated slit, parallel to the bounding surface, and wherein the second distance is in a range of 0-10 mm, and preferably in a range of 0-5 mm.

In some embodiments, the moveable element further comprises a guiding surface, which is joined with and inclined away from the support surface, and wherein the guiding surface, when the moveable element is in the first position, is arranged intermediate the support surface and the inlet port along the travel path so as to direct the leading end of the web material from the inlet port onto the support surface.

In some embodiments, at least one of the elongated seals comprises two opposing guide surfaces that extend along said one of the elongated seals and are tapered towards an elongated slit in the seal, wherein the opposing guide surfaces are arranged to guide a front end of the web material towards the elongated slit, which is adapted to receive the web material.

In some embodiments, the support surface has a width that is at 90% of a width of the web material, or is equal to or larger than the width of the web material.

In some embodiments, the moveable element is moveable between the first and second positions by rotation around a rotation axis.

In some embodiments, the moveable element comprises a rod, which is arranged for rotation within the housing and comprises an end portion which is arranged to project through a wall portion of the housing.

In some embodiments, the rotation axis is located beneath the inlet port.

In some embodiments, the rotation axis extends parallel to the inlet port.

In some embodiments, the device further comprises a manipulator, which is located outside of the housing and coupled by a linkage to the moveable element so as to move the moveable element between the first position and the second position.

In some embodiments, said arrangement is operable to reduce the presence of microorganisms on the web material by providing one or more of: heat, a disinfection agent, a sterilization agent, ultraviolet radiation, or an electron beam.

A second aspect relates to a system for manufacture of packages. The system comprises a supply device for web material; a device according to the first aspect or any of its embodiments, which device is arranged to receive the web material from the supply device and operable to reduce presence of microorganisms on the web material; and a filling machine, which is configured to receive the web material from the device and process the web material into packages.

Still other objectives, features, embodiments, and aspects, as well as additional features and advantages will appear from the following detailed description as well as from the accompanying schematic drawings.

### Brief Description of the Drawings

FIG. 1A is a schematic view of an operative system for manufacture of packages, and FIG. 1B is a perspective view of rolled-up web material for use in the system of FIG. 1A.
FIG. 2 is a section view of an example disinfection device in the operative system of FIG. 1A.
FIGS 3A-3H are section views of example disinfection devices for use in the system of FIG. 1A.
FIGS 4A-4B are perspective and section views of an example disinfection device, in which a moveable support is arranged in a service position.
FIGS 5A-5B are perspective and section views of the disinfection device of FIGS 4A-4B, in which the moveable support is arranged in a production position.
FIG. 6 corresponds to FIG. 5B and illustrates an attempt to introduce a web material into the disinfection device when the moveable support is in the production position.
FIG. 7 is a flow chart of an example method of controlling a disinfection device.

### Detailed Description

Embodiments will now be described more fully hereinafter with reference to the accompanying drawings, in which some, but not all, embodiments are shown. Indeed, the subject of the present disclosure may be embodied in many different forms and should not be construed as limited to the embodiments set forth herein; rather, these embodiments are provided so that this disclosure may satisfy applicable legal requirements.

Where possible, any of the advantages, features, functions, devices, and/or operational aspects of any of the embodiments described and/or contemplated herein may be included in any of the other embodiments described and/or contemplated herein, and/or vice versa. In addition, where possible, any terms expressed in the singular form herein are meant to also include the plural form and/or vice versa, unless explicitly stated otherwise. Accordingly, the terms "a" and/or "an" shall mean "at least one" or "one or more", even though the phrase "one or more" or "at least one" is also used herein. The terms "multiple", "plural" and "plurality" are intended to imply provision of two or more elements. The term "and/or" includes any and all combinations of one or more of the associated listed elements. Although the terms first, second, etc. may be used herein to describe various elements, these elements should not be limited by these terms. These terms are only used to distinguish one element from another. For example, a first element could be termed a second element, and, similarly, a second element could be termed a first element, without departing the scope of the present disclosure.

Well-known functions or constructions may not be described in detail for brevity and/or clarity. Unless otherwise defined, all terms (including technical and scientific terms) used herein have the same meaning as commonly understood by one of ordinary skill in the art to which this disclosure belongs.

Like reference signs refer to like elements throughout.

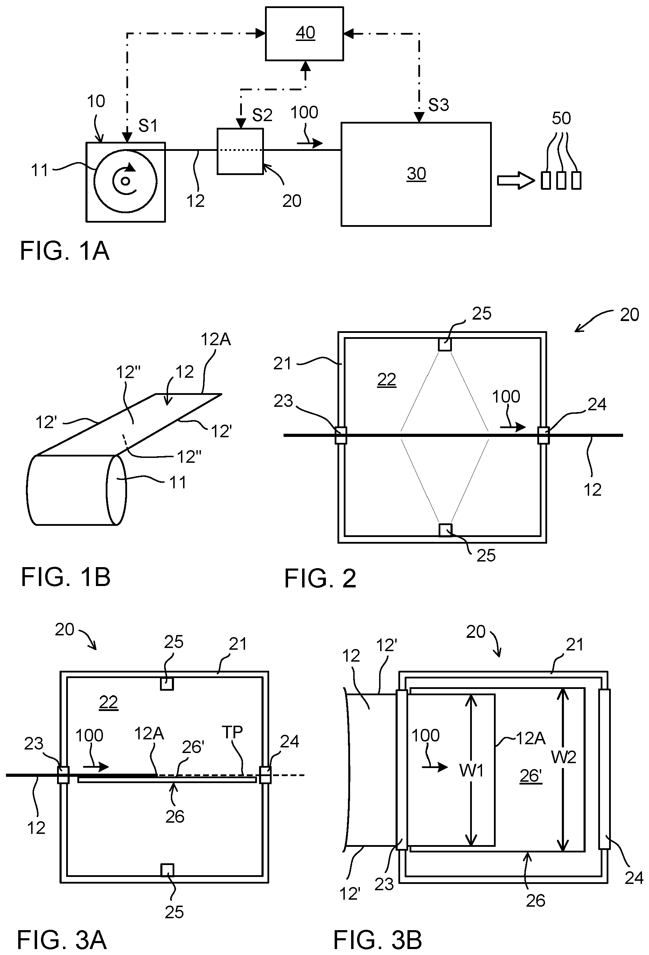

FIG. 1A is a schematic overview of an example system 1 for manufacturing packages 50. The packages 50 may contain any type of product. In the following, it is assumed that the packages 50 are filled with a food product. In the illustrated example, the manufacturing system 1 includes a web supply device 10, a device 20 for reducing presence of microorganisms, and a filling machine 30. The web supply device 10 comprises a roll 11 of web material 12, which is fed through the device 20 and into the filling machine 30. The filling machine 30 is configured to form a section of the web material 12 into a container, fill the container, and seal the filled container, to produce the packages 50. Typically, the filling machine 30 is configured to cut, fold and shape the incoming web material into containers. The filling machine 30 may of any conventional type and may be of unitary structure or a combination of physically separate units.

The web material 12 ("web") is a flat sheet of any suitable material. The present disclosure is not limited to any specific composition of the web 12. However, in many systems for manufacture of packages, the web 12 comprises a cellulose-based material, such as paper or paperboard, which may or may not be laminated with one or more layers of plastic material and/or metal, on one or both sides. The web 12 is typically provided in rolled-up form to the web supply device 10. Such a roll 11 of web material is shown in FIG. 1B. The web 12 that is withdrawn from the roll 11 has two opposing flat surfaces 12", which are joined by longitudinal edges 12'. In other words, the flat surfaces 12" are bounded by the edges 12' along the web 12.

The device 20 is configured to receive and process the web 12 to reduce presence of microorganisms thereon. As used herein, the term "microorganism" refers to any microscopic organism including but not limited to bacteria, fungi, archaea, protists, viruses, prions, etc. Depending on implementation, the device 20 may be configured to perform a disinfection or a sterilization of the web 12. As used herein, "disinfection", is a process that inactivates or destroys many but not necessarily all microorganisms, whereas "sterilization" refers to a process that removes, kills, or deactivates all microorganisms. For simplicity, the device 20 is referred to as a "disinfection device" in the following, although it may instead be used for sterilization.

One reason for installing the disinfection device 20 is to mitigate propagation of microorganisms to downstream equipment in the manufacturing system 1. This is particularly relevant in packaging of food products, where sanitary requirements are high. The disinfection device 20 need not be located upstream of the filling machine 30, as shown in FIG. 1A, but may be installed at any location within the filling machine 30. It should also be noted that the disinfection device 20 may be supplemented by further equipment for disinfection or sterilization within the filling machine 30.

The manufacturing system 1 is operated by one or more controllers, schematically represented by a control unit 40 in FIG. 1A. The control unit 40 is configured to provide control signals and receive feedback signals. These signals are schematically designated S1, S2 and S3 and represented by double-ended arrows (dot dashed) in FIG. 1A.

As indicated by an arrow 100, the web 12 is fed from the supply device 10 through the disinfection device 20 and into the filling machine 30. It is understood that the system 1 comprises one or more web feeding mechanisms, for example in the filling machine 30 and/or as a separate component.

FIG. 2 is a section view of an example disinfection device 20 during operation. The disinfection device 20 comprises a housing 21 that defines an interior space 22, in which the web 12 is processed for disinfection or sterilization. Inlet and outlet ports 23, 24 are arranged on the housing 21 to receive the web 12. The respective port 23, 24 is elongated and adapted to pass the web 12. The web 12 enters the device 20 through the inlet port 23, traverses the space 22 on a travel path, and leaves the device 20 through the outlet port 24. Typically, the web 12 is continuously fed through the device 20 in the feed direction 100 during operation of the filling machine 30. The device 20 also comprises an arrangement 25 for reducing the presence of microorganisms on the web 12. For simplicity, the arrangement 25 is denoted "microorganism mitigation arrangement" or MMA in the following. In FIG. 2, the MMA 25 is represented by two processing units, one above and one beneath the travel path of the web 12 within the space 22. The respective processing unit may be an emitter of radiation or a port for injection of a disinfection/sterilization substance. Any number of processing units may be provided within the space 22. In some embodiments, the MMA 25 is configured to subject the web 12 to ultraviolet (UV) radiation, e-beam radiation, one or more disinfectants, one or more sterilization agents, or heat, or any combination thereof. Non-limiting examples of commonly used disinfectants and sterilization agents include hydrogen peroxide, ethylene oxide, peracetic acid, formaldehyde, ozone, chlorine dioxide, etc.

Although not shown in FIG. 2, a web guiding unit may be located on the upstream side and/or on the downstream side of the disinfection device 20, to accurately position the web 12 in relation to the inlet and outlet ports 23, 24. Such web guiding units are well-known in the art.

It may also be noted that the travel path of the web 12 through the disinfection device 20 need not be horizontal but could have any direction in relation to the direction of gravity. Thus, the disinfection device 20 in FIG. 2 could have any orientation.

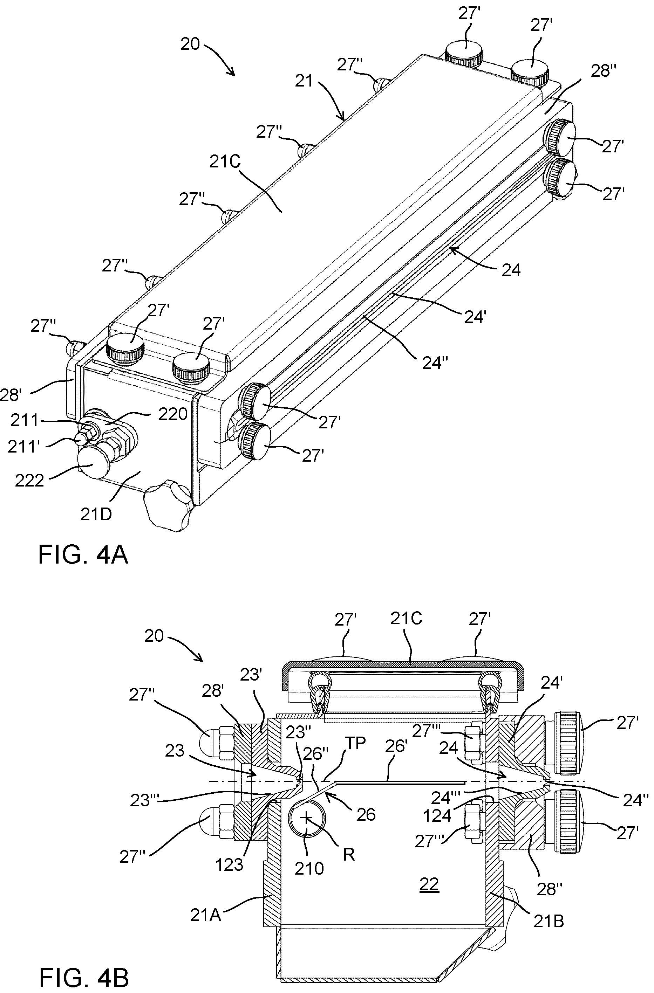

The disinfection or sterilization that is performed in the device 20 typically involve substances that are potentially harmful to humans. These substances may be provided by the MMA 25 or be generated during disinfection/sterilization. There is thus a need to mitigate uncontrolled release of substances from the space 22 in the device 20 to the surroundings. To this end, the ports 23, 24 are provided with flexible sealing elements adapted to flexingly engage the flat surfaces 12" of the web 12. A non-limiting example of sealing elements is given further below with reference to FIGS 4-6.

While the provision of ports 23, 24 on opposite sides of the device 20 allows the web12 to be simply and efficiently fed through the device 20 for disinfection/sterilization, it also causes difficulties when the web 12 is to be inserted into the device 20 to extend between the ports 23, 24. As the web 12 is extracted from the roll 11, it is likely to have some curvature in correspondence with its rolled-up form. Further, since the web 12 is not stiff, it may also be prone to twist or bend around its longitudinal direction by the action of gravity. The web 12 may also be quite wide, which makes it even more difficult to handle by operators. The insertion of the web 12 is made even more difficult by the provision of the sealing elements in the ports 23, 24. Thereby, the startup of the production line may become time consuming and industrious for the operators. Likewise, the switching of rolls during production may require significant downtime. The start-up time may be reduced by leaving the web 12 in the production line between production cycles. However, this may lead to hygienic performance issues in the production line. Also, the web 12 that was left in the production line may need to be discarded, resulting in undesirable waste of web material.

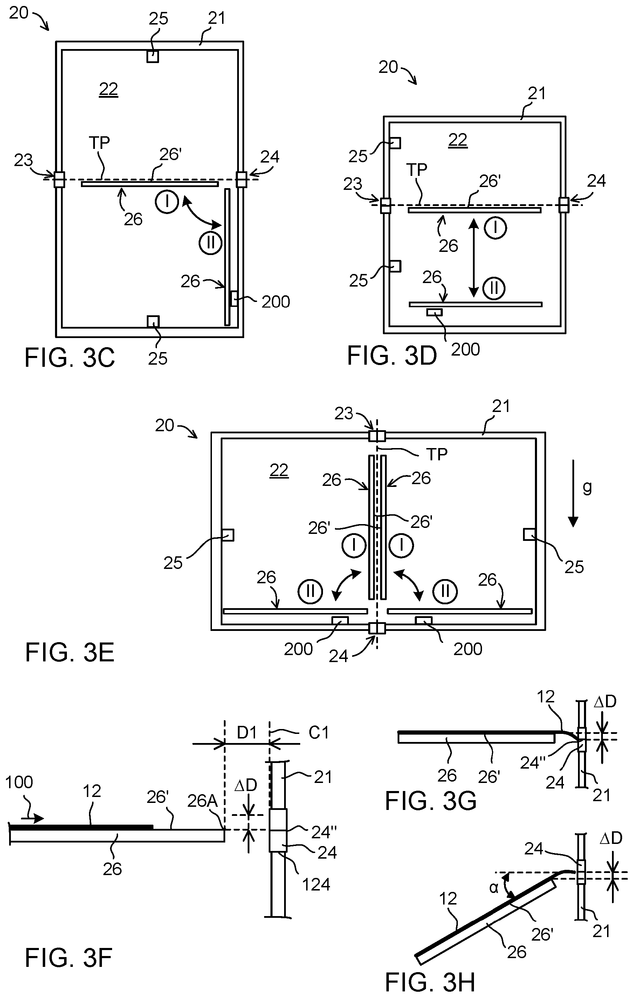

A solution to this problem is shown in FIG. 3A, which is a sectional side view of a disinfection device 20 similar to the one in FIG. 2. A supporting element 26 defines a support surface 26' for the web 12. The support surface 26' is arranged to extend along the travel path TP of the web 12 between the ports 23, 24. As seen, a leading end 12A of the web 12 will be guided along the support surface 26' towards the outlet port 24 as the web 12 is inserted through the inlet port 23 and moved in the feed direction 100 through the interior space 22. The leading end 12A is also denoted "web end" herein. By proper configuration and arrangement of the support surface 26', and possibly the outlet port 24, the web end 12A will eventually arrive at and be driven through the outlet port 24. FIG. 3B is a section view of the device in FIG. 3A, from the top of the device 20. The width of the web 20 between the edges 12' is designated by W1. The corresponding width of the support surface 26' is designated by W2. To prevent that the web 12 bends at the longitudinal edges 12', W2 should be equal to or larger than W1, as shown in FIG. 3B. However, if the web 12 has some inherent stiffness against bending, it may be sufficient for W2 to be at least 90% of W1.

The present Applicant has found that the supporting element 26 in FIGS 3A-3B is likely to impede the disinfection/sterilization of the web 12, since the supporting element 26 partly blocks the web 12. This effect remains even if the supporting element 26 is configured as a grid.

To achieve both the guiding effect and proper disinfection/sterilization, the supporting element 26 is configured as a moveable element within the housing. The moveable element 26 is also denoted "web guide" herein. The web guide 26 is arranged for movement into a first position, like in FIGS 3A-3B, and a second position, in which the web guide 26 is spaced from the travel path.

An implementation example is shown in FIG. 3C, in which the web guide 26 is arranged for rotation between the first position, designated by I, and the second position, designated by II. Position I is a service position, and position II is a production position. In position I, the support surface 26' is arranged to extend between the ports 23, 24 along the travel path TP, like the support surface 26' in FIGS 3A-3B. In position II, the support surface 26' is swung away from the travel path TP to uncover or expose the lower flat surface 12" of the web 12. In some embodiments, for example as shown in FIG. 3C, the web guide 26 is rotated by approximately 90° between the first and second positions. A rotation of the web guide 26 is simple to implement in a spaceefficient way.

Another implementation example is shown in FIG. 3D, in which the web guide 26 is instead arranged for translation between position I and position II. Position I is the same as in FIG. 3C. In position II, the support surface 26' has approximately the same orientation as in position I but is shifted away from the travel path TP.

Typically, the MMA 25 is a fixedly installed in the housing 21 and requires unrestricted access to the surfaces of the web 12 during disinfection/sterilization. For example, the MMA 25 may irradiate the web 12 from one or more specific locations, spray a fluid onto the web from one or more specific locations, or emit a vapor or aerosol for dispersion within the interior space 22. In the implementation examples of FIGS 3C-3D, the web guide 26 is arranged in position II so as to provide the MMA 25 unobscured access to the web 12. The unobscured access ensures that the MMA 25 is operable to disinfect/sterilize all parts of the web 12. The unobscured access may have different meanings depending on MMA 25. If a vapor or aerosol is dispersed within the space 22, it may be sufficient to displace the support surface 26' from the web 12 to allow the vapor/aerosol to interact with all sides of the web 12. If the web is irradiated or spayed, the web guide 26 may need to be moved out of the line of sight between the MMA 25 and the web 12.

A further implementation example is shown in FIG. 3E, in which the device 20 is provided with two web guides 26 with a respective support surface 26'. Like in FIGS 3C-3D, the respective web guide 26 has a position I, in which the web 12 is supported/guided, and a position II, in which the web 12 is fully exposed to the processing by the MMA 25. If both of the web guides 26 are moved into position I, the web 12 will be guided in a confined channel formed between the opposing support surfaces 26'. The use of two web guides 26 may serve to reduce the impact of curvature of the web 12 and improve the guiding of the web end 12A through the housing 21. It may be particularly beneficial when the travel path TP is substantially parallel to the direction of gravity g, as shown in FIG. 3E. It may be noted that the web guides 26 need not attain position I simultaneously. On the contrary, it may be beneficial to first arrange one web guide 26 in position I, insert the web 12 through the inlet port 23 onto the support surface 26', and then arrange the other web guide 26 in position I.

As shown in FIGS 3C-3E, the device may include a sensor 200, which is arranged to generate a sensor signal indicative of whether the web guide 26 is in position I or II. For example, the sensor signal may be used to indicate to an operator if the web guide 26 is in position I or II, for example on a display or by an indicator lamp. The sensor 200 may be arranged inside the housing 21, as shown, or outside of the housing 21, for example to sense the movement of a mechanical transmission coupled to the web guide 26. The sensor 20 may be a mechanical switch, or any type of proximity sensor, which may be inductive, capacitive, photoelectric, magnetic, etc. The device 20 may include one sensor 200 for detecting position I, and another sensor 200 for detecting position II. If a single sensor is used, it may be preferable to arrange the sensor 200 to detect position II, to actively confirm that the web guide 26 is moved away from the web 12 inside the housing 21.

The web guide 26 may be manually shifted between position I and position II. An example is shown in FIGS 4-6 and discussed further below. Alternatively, the device 20 may comprise an electrically operated actuator (not shown), which is mechanically coupled to the web guide 26 and operable to shift the web guide 26 between positions I and II. The actuator may be controlled by a control signal from the control unit 40 (cf. signal S2 in FIG. 1A).

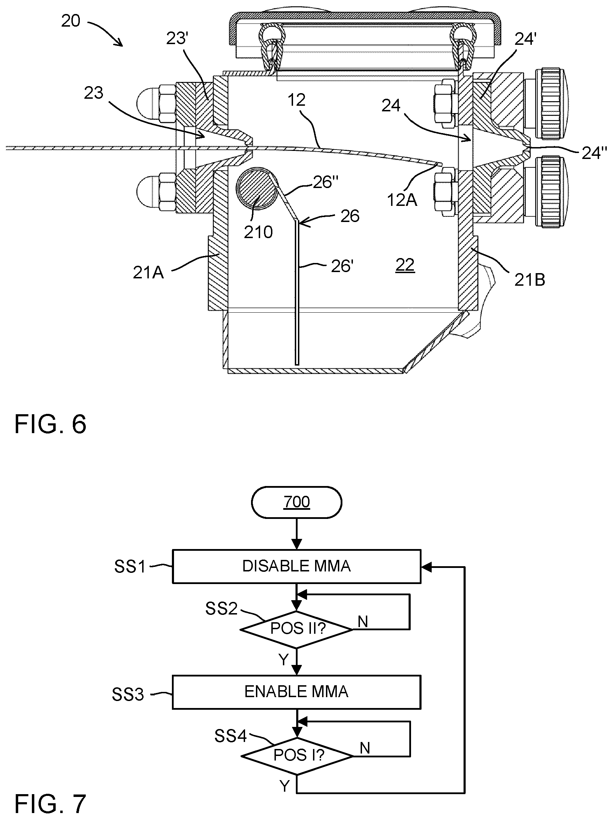

Irrespective of how the web guide 26 is shifted, manually or electrically, it may be desirable to configure the control unit 40 to operate the MMA 25 based on the sensor signal from the sensor 200. FIG. 7 is a flow chart of an example method 700 performed by the control unit 40. In step SS1, the MMA 25 is disabled and thus inoperable to perform disinfection/sterilization. This may be a default state of the MMA 25 or a state attained by the MMA 25 subject to a control signal (cf. S2 in FIG. 1A) from the control unit 40. In step SS2, the control unit 40 monitors the sensor signal to detect if position II is attained. If not, the control unit 40 returns to step SS1. When the sensor signal indicates that position II has been attained, the control unit 40 proceeds to step SS3, in which it transmits a control signal to the device 20 to enable the MMA 25. The MMA 25 is thereby operable to perform disinfection/sterilization. Step SS3 may also comprise starting the MMA 25 to perform the disinfection/sterilization. Alternatively, the MMA 25 may be manually started by an operator, for example by pushing a dedicated button on the device 20 or on the MMA 25. After step SS3, the control unit 40 proceeds to step SS4, in which it monitors the sensor signal to detect if position I is attained. When the sensor signal indicates that position I has been attained, the method returns to step SS1. It is realized that the method 700 ensures that disinfection/sterilization is only performed when the web guide 24 is displaced from the web 12.

In the examples of FIGS 3C-3E, the support surface 26' is substantially flat. This may be advantageous to ensure well-controlled guiding of the web 12 at low friction. However, it is conceivable that the support surface 26' is curved, for example convex, in relation to the travel path TP when the web guide 26 is in position I. In another alternative, the surface 26' is corrugated, for example by ridges extending on the surface 26' along travel path TP when the web guide 26 is in position I.

The support surface 26' may be solid or include openings or holes. For example, the support surface 26' may be formed as a grid or mesh.

In the examples of FIGS 3C-3E, the support surface 26' is arranged to be parallel with the elongated extent of the ports 23, 24, when the web guide 26 is in position I. For example, the support surface 26' may be parallel to an elongated slit (cf. 24" in FIGS 4-6) of the outlet port 24. This will facilitate the guiding of the web end 12A through the housing 21 and into the outlet port 24.

FIG. 3F is a side view of a web guide 26 in relation to an outlet port 24 and serves to illustrate some design parameters for the device 20. In the illustrated example, the port 24 comprises an elongated seal 24, which is fitted to cover an access opening 124 in the housing 21. The seal 24 comprises a slit 24" that extends along the elongated seal 24. The slit 24" is arranged to receive the web 12. As indicated by a dashed line, a bounding surface C1 is defined by the port 24 inside the housing. The bounding surface C1 is a geometric plane, which coincides with the innermost point on the port 24. In the illustrated example, the bounding surface C1 is defined by the innermost portion of the seal 24. In other embodiments, the bounding surface C1 may instead be defined by the housing material around the access opening 124.

One design parameter is represented as D1 and is the perpendicular distance from the bounding surface C1 to a front end 26A of the support surface 26'. It may be noted that the support surface 26' may but need not have a rectangular contour in plan view (cf. FIG. 3B). For example, the front end 26A may have an irregular contour in plan view. As used herein, the front end 26A refers to the portion of the support surface 26' that is located closest to the bounding surface C1 in position I. In some embodiments, the distance D1 is in the range of 0-50 mm. This has been found result in proper guiding of the web 12 into the outlet port 24. In some embodiments, the lower limit of D1 is 0.5 mm to reduce the impact of tolerances and/or dimensional variations with temperature. In some embodiments, the upper limit of D1 is 40 mm, 30 mm, or 20 mm to further improve the guiding properties, for example to handle webs 12 with larger curvature at the web end 12A.

Another design parameter is represented as ΔD and is the distance, measured parallel to the bounding surface C1, from the front end 26A to the elongated slit 24". In some embodiments, ΔD is approximately zero. This may be appropriate if the web 12 is known to lack curvature at the web end 12A. In some embodiments, ΔD is larger than zero but smaller than about 5 mm or 10 mm to handle curvature of the web 12. The impact of curvature is exemplified in FIG. 3G, in which the support surface 26' is arranged approximately perpendicularly to the bounding surface C1, and the front end 26A is displaced by ΔD from the slit 24" to steer the web end 12A onto the slit 24". An alternative or supplementary way of handling curvature is shown in FIG., 3H, in which the support surface 26' is inclined away from the slit 24" by an angle α, which set in view of the curvature so that the web end 12A is steered onto the slit 24".

The use of the design parameters D1, ΔD and α as described in the foregoing are examples of a general concept of configuring the web guide 26 based on a known (nominal) curvature of the web 12, so as to direct the web end 12A onto the outlet port 24 when the web guide 26 is in position I. As exemplified above, the web guide 26 may be configured to arrange the support surface 26" with an inclination and/or a shift in relation to the elongated slit 24" in the outlet port 24 to achieve this objective.

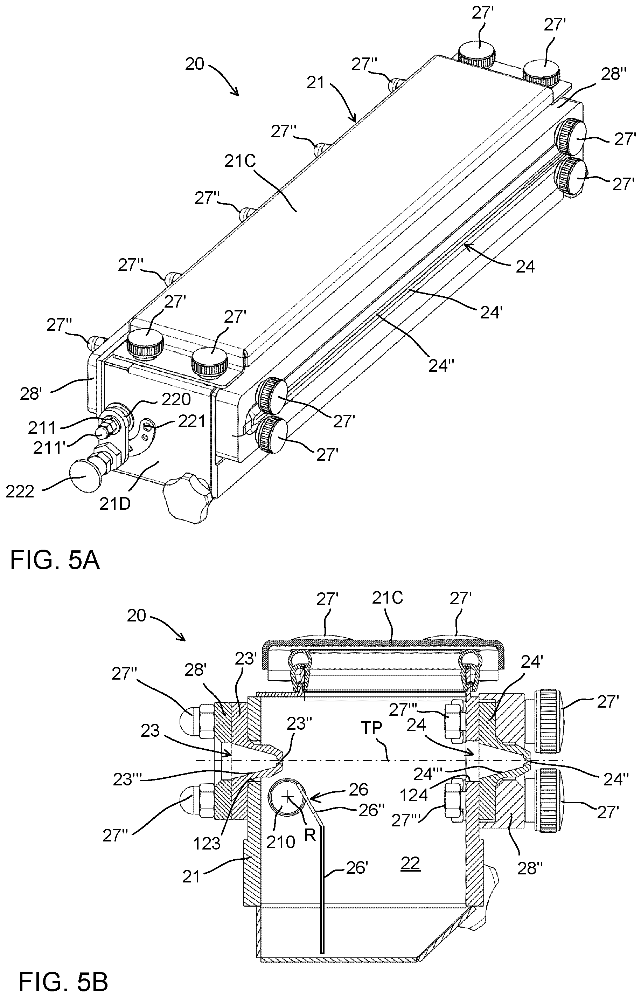

FIGS 4A-4B show an example disinfection device 20, which has the web guide 26 in the service position (position I), and FIGS 5A-5B show the disinfection device 20 with the web guide 26 in the production position (position II). As seen in the perspective view of FIG. 4A, the disinfection device 20 is elongated and has a housing 21 in the general shape of a rectangular cuboid. The housing 21 comprises wall portions, some of which are part of a fixed frame structure and some being releasably attached to the frame structure. In the illustrated example, the frame structure comprises an inlet wall 21A and an outlet wall 21B, which define an inlet access opening 123 and an outlet access opening 124, respectively. A top lid 21C is releasably attached to the frame structure by bolts with knob handles 27', which are configured to facilitate manipulation by hand. The knob handles 27' thus form quick-release connectors that are simple to remove when the device 20 needs to be dismantled for cleaning or other maintenance. Any other type of quick-release connector may be used.

An inlet seal 23' is arranged to cover the inlet access opening 123 and project into the housing 21. An inlet mounting plate 28' defines an opening that conforms in shape and position with the inlet access opening 123. The inlet seal 24' is sandwiched between the inlet mounting plate 28' and the inlet wall 21A. The inlet mounting plate 28' is attached to the inlet wall 21A by fasteners 27", which in this example are nuts screwed onto threaded ends (not shown) that are arranged to project from the inlet wall 21A through mounting holes (not shown) in the mounting plate 28' and in the seal 23'. An outlet seal 24' is arranged to cover the outlet access opening 124 and project out of the housing 21. An outlet mounting plate 28" defines an opening that conforms in shape and position with the outlet opening 124. The outlet seal 24' is sandwiched between the mounting plate 28" and the outlet wall 21B. The outlet mounting plate 28" is attached to the outlet wall 21B by fasteners, in this example bolts with knob handles 27'. The bolts extend through mounting holes (not shown) in the mounting plate 28", in the seal 24' and in the outlet wall 21B into engagement with nuts 27‴ on the inside of the housing 21.

The inlet seal 23' and the access opening 123 are part of the inlet port 23, and the outlet seal 24' and the access opening 124 are part of the outlet port 24. The respective seal 23', 24' comprises an elongate slit 23", 24".

As seen in the section views of FIGS 4B and 5B, the web guide 26 is rotatably arranged in the housing 21 and moveable between position I (FIG. 4B) and position II (FIG. 5B) by rotation around a rotation axis R. The rotation axis R is located beneath the inlet port 23. This location has the advantage of ensuring that the free end of the web guide 26 points away from the inlet port 23, which eliminates the risk that the web end 12A falls beneath the web guide 26 upon entry through the inlet port 23.

It is also understood from FIGS 4B and 5B that the rotation axis R extends parallel to the inlet port 23. This will facilitate the construction of the web guide 26.

As shown in FIGS 4A and 5B, the web guide 26 may be configured to define a guiding surface 26" adjacent to the support surface 26'. The guiding surface 26" is joined with and inclined away from the support surface 26'. As shown in FIG. 4B, when the web guide 26 is in position I, the guiding surface 26" is arranged intermediate the support surface 26' and the inlet port 23, to bridge the gap between them along the travel path TP. The inclined guiding surface 26" is capable of directing the web end 12A from the inlet port 23 onto the support surface 26' even if the web end 12A has significant curvature.

In the illustrated example, the web guide 26 includes a rod 210, which extends along the elongated extent of the housing 21 and is arranged for rotation within the housing 21. The rod 210 is an integral part of the web guide 26. An end portion 211 of the rod 210 is arranged to project through an end wall 21D of the housing 21. A linkage 220 is keyed onto the projecting end portion 211 and attached by a fastener 211' (here, a nut). A manipulator 222 is arranged on the linkage 220 and operable to rotate the web guide 26 within the housing 21 into position I and position II. A locking element 221 is arranged on the end wall 21D and has openings for engagement with a locking pin of the manipulator 222. The locking pin is biased towards the end wall 21D. To move the web guide 26 inside the housing, the operator pulls the manipulator 222 away from the end wall 21D and swings the manipulator 222 to the right or the left. When the operator releases the manipulator 22 in proper alignment with an opening in the locking element 221, the locking pin snaps into engagement with this opening, and the web guide 26 is locked in position.

It may be noted in FIGS 4A and 5A that the linkage 220 is arranged to have the same orientation as the web guide 26, which may serve to mitigate operator errors.

In the section views of FIGS 4B and 5B, it is seen that the seals 23', 24' are configured to project through the access openings 123, 124 of the housing 21. The inlet seal 23' is configured to project into the housing 21, and the outlet seal 24' is configured to project out of the housing 21. Thereby, the seals 23', 24' are arranged project in the feed direction of the web 12 along the travel path TP through the housing. The projecting shape of the seals 23', 24' makes it possible to configure the respective seal 23', 24' with two opposing guide surfaces 23‴, 24‴ that extend along the elongated seal 23', 24' and taper towards the slit 23", 24". The guide surfaces 23'", 24‴ face in a direction opposite to the feed direction of the web 12 through the housing 21 (cf. 100 in FIG. 1A). This configuration of the seals 23', 24' may facilitate the task of inserting the web end 12A into the respective slit 23', 24'. The web end 12A will be guided through contact with the guide surfaces 23‴, 24‴ to and through the respective slit 23', 24'.

FIG. 6 is a section view of the device 20 in FIGS 4-5. In FIG. 6, the web guide 26 is set in position II while the web 12 is inserted into the housing. As seen, even for a moderate curvature of the web 12, the web 12A is likely to miss the outlet port 24. The bending of the web 12 may be further aggravated by the action of gravity, depending on the orientation of the housing in relation to gravity. It may also be noted that, in the absence of the support surface 26', gravity may also cause the web 12 to twist around its longitudinal axis, resulting in the web end 12A being non-parallel to the slit 24' of the outlet port 24. All of these problems are mitigated by shifting the web guide 26 into position I.

## Claims

1. A device for reducing presence of microorganism on a web material (12), said device comprising:
a housing (21) that defines an interior space (22),
an inlet port (23) and an outlet port (24), which are arranged on the housing (21) to allow the web material (12) to move along a travel path (TP) through the interior space (22) between the inlet port (23) and the outlet port (24), wherein the inlet and outlet ports (23, 24) are elongated and comprise a respective elongated seal (23', 24') for flexing engagement with the web material (12), and
an arrangement (25) for reducing the presence of microorganisms on the web material (12) within the interior space (22),
**characterised in that** said device further comprising:
a moveable element (26) comprising a support surface (26'), wherein the moveable element (26) is arranged within the housing (21) for movement into a first position (I), in which the support surface (26') is arranged along the travel path (TP) so as to direct a leading end (12A) of the web material (12) from the inlet port (23) towards the outlet port (24), and a second position (II), in which the support surface (26') is spaced from the travel path (TP).

2. The device of claim 1, wherein the support surface (26'), when the moveable element (26) is in the second position (II), is spaced from the travel path (TP) so as provide said arrangement (25), which is fixedly arranged in the housing (21), unobscured access to the web material (12) to reduce the presence of microorganisms on the web material (12).

3. The device of claim 1 or 2, further comprising a sensor (200), which is arranged to generate a signal indicative of whether the moveable element (26) is in the first position (I) or the second position (II).

4. The device of claim 3, further comprising a control unit (40), which is configured to operate said arrangement (25) based on the signal from the sensor (200).

5. The device of claim 4, wherein the control unit (40) is configured to disable said arrangement (25) when the moveable element (26) is in the first position (I) and enable said arrangement (25) when the moveable element (26) is in the second position (II).

6. The device of any preceding claim, wherein the outlet port (24) comprises an access opening (124) in the housing (1), and the elongated seal (24'), which is arranged to cover the access opening (124), wherein the access opening (124) and/or the elongated seal (24') defines a bounding surface (C1) of the outlet port (24) inside the housing (21), wherein the support surface (26'), when the moveable element (26) is in the first position (I), is inclined and/or shifted in relation to the bounding surface (C1), based on a predefined curvature of the web material (12), so as to direct the leading end (12A) of the web material (12) to outlet port (24).

7. The device of claim 6, wherein a front end (26A) of the support surface (26') is located at the outlet port (24) when the moveable element (26) is in the first position (I), and wherein a first distance (D1) between the front end (26A) and the bounding surface (C1), perpendicular to the bounding surface (C1), is in a range of 0-50 mm, and preferably in a range of 0.5-40 mm.

8. The device of claim 7, wherein the elongated seal (24) defines an elongated slit (24") for receiving the web material (2), wherein the front end (26A) of the support surface (26'), when the moveable element (26) is in the first position (I), is located a second distance (ΔD) from the elongated slit (24"), parallel to the bounding surface (C1), and wherein the second distance (ΔD) is in a range of 0-10 mm, and preferably in a range of 0-5 mm.

9. The device of any preceding claim, wherein the moveable element (26) further comprises a guiding surface (26"), which is joined with and inclined away from the support surface (26'), and wherein the guiding surface (26"), when the moveable element (26) is in the first position (I), is arranged intermediate the support surface (26') and the inlet port (23) along the travel path (TP) so as to direct the leading end (12A) of the web material (12) from the inlet port (23) onto the support surface (26').

10. The device of any preceding claim, wherein at least one of the elongated seals (23, 24) comprises two opposing guide surfaces (23"', 24‴) that extend along said one of the elongated seals (23, 24) and are tapered towards an elongated slit (23", 24") in the seal (23, 24), wherein the opposing guide surfaces (23"', 24"') and arranged to guide a front end (12A) of the web material (12) towards the elongated slit (23", 24"), which is adapted to receive the web material (12).

11. The device of any preceding claim, wherein the support surface (26') has a width (W2) that is at 90% of a width (W1) of the web material (12), or is equal to or larger than the width (W1) of the web material (12).

12. The device of any preceding claim, wherein the moveable element (26) is moveable between the first and second positions (I, II) by rotation around a rotation axis (R).

13. The device of claim 12, wherein the moveable element (26) comprises a rod (210), which is arranged for rotation within the housing (21) and comprises an end portion (211) which is arranged to project through a wall portion (21D) of the housing (21).

14. The device of claim 12 or 13, wherein the rotation axis (R) is located beneath the inlet port (23).

15. A system for manufacture of packages, said system comprising:
a supply device (10) for web material (12),
a device (20) according to any one of claims 1-14, which is arranged to receive the web material (12) from the supply device (10) and operable to reduce presence of microorganisms on the web material (12), and
a filling machine (30), which is configured to receive the web material (12) from the device (20) and process the web material (12) into packages (50).

## Patentansprüche

1. Vorrichtung zum Verringern des Vorhandenseins von Mikroorganismen auf einem Bahnmaterial (12), wobei die Vorrichtung umfasst:
ein Gehäuse (21), das einen Innenraum (22) definiert,
eine Einlassöffnung (23) und eine Auslassöffnung (24), die an dem Gehäuse (21) angeordnet sind, um Bewegung des Bahnmaterials (12) entlang eines Bewegungswegs (TP) durch den Innenraum (22) zwischen der Einlassöffnung (23) und der Auslassöffnung (24) zu ermöglichen, wobei die Einlass- und die Auslassöffnung (23, 24) langgestreckt sind und eine entsprechende langgestreckte Dichtung (23', 24') zum Biegeeingriff mit dem Bahnmaterial (12) umfassen, und
eine Anordnung (25) zum Verringern des Vorhandenseins von Mikroorganismen auf dem Bahnmaterial (12) in dem Innenraum (22),
**dadurch gekennzeichnet, dass** die Vorrichtung ferner umfasst:
ein bewegliches Element (26), das eine Trägerfläche (26') umfasst, wobei das bewegliche Element (26) in dem Gehäuse (21) zur Bewegung in eine erste Position (I) angeordnet ist, in der die Trägerfläche (26') entlang des Bewegungswegs (TP) angeordnet ist, um ein Vorderende (12A) des Bahnmaterials (12) von der Einlassöffnung (23) zu der Auslassöffnung (24) zu führen, und eine zweite Position (II), in der die Trägerfläche (26') von dem Bewegungsweg (TP) beabstandet ist.

2. Vorrichtung nach Anspruch 1, wobei die Trägerfläche (26'), wenn sich das bewegliche Element (26) in der zweiten Position (II) befindet, von dem Bewegungsweg (TP) beabstandet ist, um für die Anordnung (25), die fest in dem Gehäuse (21) angeordnet ist, ungehinderten Zugang zu dem Bahnmaterial (12) bereitzustellen, um das Vorhandensein von Mikroorganismen auf dem Bahnmaterial (12) zu verringern.

3. Vorrichtung nach Anspruch 1 oder 2, ferner umfassend einen Sensor (200), der dafür angeordnet ist, ein Signal zu erzeugen, das anzeigt, ob sich das bewegliche Element (26) in der ersten Position (I) oder der zweiten Position (II) befindet.

4. Vorrichtung nach Anspruch 3, ferner umfassend eine Steuereinheit (40), die dafür gestaltet ist, die Anordnung (25) auf der Grundlage des Signals von dem Sensor (200) zu betreiben.

5. Vorrichtung nach Anspruch 4, wobei die Steuereinheit (40) dafür gestaltet ist, die Anordnung (25) zu deaktivieren, wenn sich das bewegliche Element (26) in der ersten Position (I) befindet, und die Anordnung (25) zu aktivieren, wenn sich das bewegliche Element (26) in der zweiten Position (II) befindet.

6. Vorrichtung nach einem der vorstehenden Ansprüche, wobei die Auslassöffnung (24) eine Zugangsöffnung (124) in dem Gehäuse (1) und die langgestreckte Dichtung (24'), die angeordnet ist, die Zugangsöffnung (124) abzudecken, umfasst, wobei die Zugangsöffnung (124) und/oder die langgestreckte Dichtung (24') eine Begrenzungsfläche (C1) der Auslassöffnung (24) in dem Gehäuse (21) definieren, wobei die Trägerfläche (26'), wenn sich das bewegliche Element (26) in der ersten Position (I) befindet, relativ zu der Begrenzungsfläche (C1) bezogen auf eine vorgegebene Krümmung des Bahnmaterials (12) geneigt und/oder verschoben ist, um das Vorderende (12A) des Bahnmaterials (12) zu der Auslassöffnung (24) zu führen.

7. Vorrichtung nach Anspruch 6, wobei ein vorderes Ende (26A) der Trägerfläche (26') an der Auslassöffnung (24) angeordnet ist, wenn sich das bewegliche Element (26) in der ersten Position (I) befindet, und wobei ein erster Abstand (D1) zwischen dem vorderen Ende (26A) und der Begrenzungsfläche (C1) senkrecht zu der Begrenzungsfläche (C1) in einem Bereich von 0-50 mm und vorzugsweise in einem Bereich von 0,5-40 mm liegt.

8. Vorrichtung nach Anspruch 7, wobei die langgestreckte Dichtung (24) einen langgestreckten Schlitz (24") zum Aufnehmen des Bahnmaterials (2) definiert, wobei sich das vordere Ende (26A) der Trägerfläche (26'), wenn sich das bewegliche Element (26) in der ersten Position (I) befindet, in einem zweiten Abstand (Δd) von dem langgestreckten Schlitz (24") parallel zu der Begrenzungsfläche (C1) angeordnet ist, und wobei der zweite Abstand (Δd) in einem Bereich von 0-10 mm und vorzugsweise in einem Bereich von 0-5 mm liegt.

9. Vorrichtung nach einem der vorstehenden Ansprüche, wobei das bewegliche Element (26) ferner eine Führungsfläche (26") umfasst, die mit der Trägerfläche (26') verbunden ist und davon weg geneigt ist, und wobei die Führungsfläche (26"), wenn sich das bewegliche Element (26) in der ersten Position (I) befindet, zwischen der Trägerfläche (26') und der Einlassöffnung (23) entlang des Bewegungswegs (TP) angeordnet ist, um das Vorderende (12A) des Bahnmaterials (12) von der Einlassöffnung (23) auf die Trägerfläche (26') zu führen.

10. Vorrichtung nach einem der vorstehenden Ansprüche, wobei wenigstens eine der langgestreckten Dichtungen (23, 24) zwei gegenüberliegende Führungsflächen (23"', 24"') umfasst, die entlang der einen der langgestreckten Dichtungen (23, 24) verlaufen und sich zu einem langgestreckten Schlitz (23", 24") in der Dichtung (23, 24) verjüngen, wobei die gegenüberliegenden Führungsflächen (23"', 24"') angeordnet sind, ein vorderes Ende (12A) des langgestreckten Bahnmaterials (12 in Richtung zu dem langgestreckten Schlitz (23", 24"), der zur Aufnahme des Bahnmaterials (12) gestaltet ist, zu führen.

11. Vorrichtung nach einem der vorstehenden Ansprüche, wobei die Trägerfläche (26') eine Breite (W2) aufweist, die bei 90 % einer Breite (W1) des Bahnmaterials (12) liegt oder gleich oder größer als die Breite (W1) des Bahnmaterials (12) ist.

12. Vorrichtung nach einem der vorstehenden Ansprüche, wobei das bewegliche Element (26) durch Drehung um eine Drehachse (R) zwischen der ersten und der zweiten Position (I, II) beweglich ist.

13. Vorrichtung nach Anspruch 12, wobei das bewegliche Element (26) eine Stange (210) umfasst, die zur Drehung innerhalb des Gehäuses (21) angeordnet ist und einen Endabschnitt (211) umfasst, der angeordnet ist, durch einen Wandabschnitt (21D) des Gehäuses (21) zu ragen.

14. Vorrichtung nach Anspruch 12 oder 13, wobei die Drehachse (R) unterhalb der Einlassöffnung (23) angeordnet ist.

15. System zur Herstellung von Verpackungen, wobei das System umfasst:
eine Zufuhrvorrichtung (10) für Bahnmaterial (12),
eine Vorrichtung (20) nach einem der Ansprüche 1-14, die dafür eingerichtet ist, das Bahnmaterial (12) von der Zufuhrvorrichtung (10) aufzunehmen, und funktionsfähig ist, das Vorhandensein von Mikroorganismen auf dem Bahnmaterial (12) zu verringern, und
eine Füllmaschine (30), die dafür gestaltet ist, das Bahnmaterial (12) von der Vorrichtung (20) aufzunehmen und das Bahnmaterial (12) zu Verpackungen (50) zu verarbeiten.

## Revendications

1. Dispositif destiné à réduire la présence de microorganismes sur un matériau en bande (12), ledit dispositif comprenant :
un boîtier (21) qui définit un espace intérieur (22),
un orifice d'entrée (23) et un orifice de sortie (24), qui sont disposés sur le boîtier (21) pour permettre au matériau en bande (12) de se déplacer le long d'un chemin de déplacement (TP) à travers l'espace intérieur (22) entre l'orifice d'entrée (23) et l'orifice de sortie (24), les orifices d'entrée et de sortie (23, 24) étant allongés et comprenant un joint d'étanchéité allongé respectif (23', 24') destiné à entrer en prise souple avec le matériau en bande (12), et
un agencement (25) destiné à réduire la présence de microorganismes sur le matériau en bande (12) dans l'espace intérieur (22),
**caractérisé en ce que** ledit dispositif comprend en outre :
un élément mobile (26) comprenant une surface de support (26'), l'élément mobile (26) étant disposé à l'intérieur du boîtier (21) pour se déplacer jusqu'à une première position (I) dans laquelle la surface de support (26') est disposée le long du chemin de déplacement (TP) de manière à acheminer une extrémité de tête (12A) du matériau en bande (12) depuis l'orifice d'entrée (23) vers l'orifice de sortie (24), et une deuxième position (II) dans laquelle la surface de support (26') est espacée du chemin de déplacement (TP).

2. Dispositif de la revendication 1, dans lequel la surface de support (26'), quand l'élément mobile (26) se trouve dans la deuxième position (II), est espacé du chemin de déplacement (TP) de manière à fournir audit agencement (25), qui est disposé de façon fixe dans le boîtier (21), un accès dégagé au matériau en bande (12) pour réduire la présence de microorganismes sur le matériau en bande (12).

3. Dispositif de la revendication 1 ou 2, comprenant en outre un capteur (200), qui est agencé pour générer un signal indiquant si l'élément mobile (26) se trouve dans la première position (I) ou la deuxième position (II).

4. Dispositif de la revendication 3, comprenant en outre une unité de commande (40), qui est conçue pour faire fonctionner ledit agencement (25) sur la base du signal provenant du capteur (200).

5. Dispositif de la revendication 4, dans lequel l'unité de commande (40) est conçue pour désactiver ledit agencement (25) quand l'élément mobile (26) se trouve dans la première position (I) et activer ledit agencement (25) quand l'élément mobile (26) se trouve dans la deuxième position (II).

6. Dispositif d'une quelconque revendication précédente, dans lequel l'orifice de sortie (24) comprend une ouverture d'accès (124) dans le boîtier (1), et le joint d'étanchéité allongé (24'), qui est agencé pour couvrir l'ouverture d'accès (124), dans lequel l'ouverture d'accès (124) et/ou le joint d'étanchéité allongé (24') définissent une surface de délimitation (C1) de l'orifice de sortie (24) à l'intérieur du boîtier (21), dans lequel la surface de support (26'), quand l'élément mobile (26) se trouve dans la première position (I), est inclinée et/ou décalée par rapport à la surface de délimitation (C1), en fonction d'une courbure prédéfinie du matériau en bande (12), de manière à acheminer l'extrémité de tête (12A) du matériau en bande (12) jusqu'à l'orifice de sortie (24).

7. Dispositif de la revendication 6, dans lequel une extrémité avant (26A) de la surface de support (26') se situe à l'orifice de sortie (24) quand l'élément mobile (26) se trouve dans la première position (I), et dans lequel une première distance (D1) entre l'extrémité avant (26A) et la surface de délimitation (C1), perpendiculairement à la surface de délimitation (C1), se situe dans une plage de 0 - 50 mm, et de préférence dans une plage de 0,5 - 40 mm.

8. Dispositif de la revendication 7, dans lequel le joint d'étanchéité allongé (24) définit une fente allongée (24") destinée à recevoir le matériau en bande (2), dans lequel l'extrémité avant (26A) de la surface de support (26'), quand l'élément mobile (26) se trouve dans la première position (I), se situe à une deuxième distance (ΔD) de la fente allongée (24"), parallèlement à la surface de délimitation (C1), et dans lequel la deuxième distance (ΔD) se situe dans une plage de 0 - 10 mm, et de préférence dans une plage de 0 - 5 mm.

9. Dispositif d'une quelconque revendication précédente, dans lequel l'élément mobile (26) comprend en outre une surface de guidage (26"), qui est reliée à la surface de support (26') et inclinée par rapport à celle-ci, et dans lequel la surface de guidage (26"), quand l'élément mobile (26) se trouve dans la première position (I), est disposée entre la surface de support (26') et l'orifice d'entrée (23) le long du chemin de déplacement (TP) de manière à acheminer l'extrémité de tête (12A) du matériau en bande (12) depuis l'orifice d'entrée (23) sur la surface de support (26').

10. Dispositif d'une quelconque revendication précédente, dans lequel au moins un des joints d'étanchéité allongés (23, 24) comprend deux surfaces de guidage opposées (23‴, 24"') qui s'étendent le long dudit un des joints d'étanchéité allongés (23, 24) et sont coniques vers une fente allongée (23", 24") dans le joint d'étanchéité (23, 24), dans lequel les surfaces de guidage opposées (23"', 24"') et agencées pour guider une extrémité avant (12A) du matériau en bande (12) vers la fente allongée (23", 24"), qui est adaptée pour recevoir le matériau en bande (12).

11. Dispositif d'une quelconque revendication précédente, dans lequel la surface de support (26') possède une largeur (W2) qui représente 90 % d'une largeur (W1) du matériau en bande (12), ou est supérieure ou égale à la largeur (W1) du matériau en bande (12).

12. Dispositif d'une quelconque revendication précédente, dans lequel l'élément mobile (26) est mobile entre la première et la deuxième position (I, II) par rotation autour d'un axe de rotation (R).

13. Dispositif de la revendication 12, dans lequel l'élément mobile (26) comprend une tige (210), qui est agencée pour tourner à l'intérieur du boîtier (21) et comprend une partie d'extrémité (211) qui est agencée pour faire saillie à travers une paroi latérale (21D) du boîtier (21).

14. Dispositif de la revendication 12 ou 13, dans lequel l'axe de rotation (R) se situe sous l'orifice d'entrée (23).

15. Système de fabrication d'emballages, ledit système comprenant :
un dispositif d'alimentation (10) en matériau en bande (12),
un dispositif (20) selon l'une quelconque des revendications 1 à 14, qui est agencé pour recevoir le matériau en bande (12) provenant du dispositif d'alimentation (10) et utilisable pour réduire la présence de microorganismes sur le matériau en bande (12), et
une machine de remplissage (30), qui est conçue pour recevoir le matériau en bande (12) provenant du dispositif (20) et transformer le matériau en bande (12) en emballages (50).
